(19)

**Europäisches Patentamt**
**European Patent Office**
**Office européen des brevets**

(11) **EP 4 744 772 A1**

(12) **EUROPEAN PATENT APPLICATION**
published in accordance with Art. 153(4) EPC

(43) Date of publication:
**20.05.2026 Bulletin 2026/21**

(21) Application number: **24865814.8**

(22) Date of filing: **10.09.2024**

(51) International Patent Classification (IPC):
*B01J 35/30* (2024.01)     *B01J 35/61* (2024.01)
*B01J 23/78* (2006.01)     *B01J 37/02* (2006.01)
*C07C 1/12* (2006.01)     *C10G 2/00* (2006.01)

(52) Cooperative Patent Classification (CPC):
**B01J 23/78; B01J 35/30; B01J 35/61; B01J 37/02;**
**C07C 1/12; C10G 2/00**

(86) International application number:
**PCT/KR2024/013722**

(87) International publication number:
**WO 2025/058375 (20.03.2025 Gazette 2025/12)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **15.09.2023 KR 20230123490**

(71) Applicant: **LG Chem, Ltd.**
**Seoul 07336 (KR)**

(72) Inventors:
• **KIM, Eui Tae**
**Daejeon 34122 (KR)**
• **LEE, Eun Jeong**
**Daejeon 34122 (KR)**

• **PARK, Sung Eun**
**Daejeon 34122 (KR)**
• **LEE, Geun Sung**
**Daejeon 34122 (KR)**
• **JOE, Je Mee**
**Daejeon 34122 (KR)**
• **PARK, Seung Won**
**Daejeon 34122 (KR)**
• **KIM, Won Hee**
**Daejeon 34122 (KR)**
• **KIM, Ki Hwan**
**Daejeon 34122 (KR)**
• **LEE, Yong Hee**
**Daejeon 34122 (KR)**

(74) Representative: **Hoffmann Eitle**
**Patent- und Rechtsanwälte PartmbB**
**Arabellastraße 30**
**81925 München (DE)**

(54) **CARBON DIOXIDE CONVERSION CATALYST AND METHOD FOR MANUFACTURING SAME**

(57)     The present invention relates to a catalyst for converting carbon dioxide, comprising a protective layer formed on the surface thereof, wherein by controlling the amount of coverage of the catalyst particle surface by the protective layer, high selectivity for high-carbon-number hydrocarbons having 5 or more carbon atoms, which have high value in carbon dioxide conversion reactions, can be achieved while maintaining the conversion rate of carbon dioxide at high levels.

FIG. 1

| Comparative Example 4 | | Example 4-3 | |
|---|---|---|---|
| Before heat treatment | 700°C 2h | Before heat treatment | 700°C 2h |

Description

[Technical Field]

CROSS-REFERENCE TO RELATED APPLICATION

This application claims the benefit of priority to Korean Patent Application No. 10-2023-0123490 filed on September 15, 2023, the disclosure of which is incorporated herein by reference in its entirety.

Technical Field

The present invention relates to a novel carbon dioxide conversion catalyst capable of increasing the selectivity for hydrocarbons having 5 or more carbon atoms in a reaction of converting carbon dioxide into hydrocarbons, and a manufacturing method thereof.

[Background Art]

Carbon dioxide accounts for a large proportion of greenhouse gases, and thus, technologies for reducing the amount of carbon dioxide in the atmosphere are being studied in various ways. Typically, carbon capture and storage (CCS) technologies that capture and store carbon dioxide, and carbon capture and utilization (CCU) technologies that capture carbon dioxide and then utilize it in other fields are being studied in various ways.

Among the two categories of carbon dioxide treatment technologies, a representative technology among the CCU technologies that can utilize captured carbon dioxide beyond simple storage is a technology for producing economically valuable hydrocarbons from the captured carbon dioxide. Methods for producing hydrocarbons from carbon dioxide include electrochemical, photochemical, and thermochemical methods. However, the electrochemical and photochemical methods are difficult to produce long-chain hydrocarbons and have low conversion rates of carbon dioxide, so more in-depth research is needed until they can be applied to actual industrial fields. On the other hand, the thermochemical method is a method for producing various hydrocarbon gases from carbon dioxide using various catalysts at high temperatures and under a hydrogen atmosphere, and is currently the most widely used method because the conversion rate of carbon dioxide is high compared to the two methods described above.

It is known that a reaction for producing hydrocarbons from carbon dioxide is generally composed of two consecutive reactions. The first reaction is a reaction in which carbon dioxide is converted into carbon monoxide through a reverse water gas shift (RWGS) reaction, and the second reaction is a reaction in which the carbon monoxide produced through the first reaction is converted into hydrocarbons through a Fischer-Tropsch reaction. The first reaction is an endothermic reaction, whereas the second reaction is an exothermic reaction. A large amount of energy must be supplied in order to efficiently perform the first reaction, but in the second reaction, the reaction heat must be quickly removed to prevent sintering and activity deterioration of the catalyst, so it is not easy to efficiently operate the reaction process due to the characteristics of such a two-stage reaction. In particular, if the reaction heat generated during the second reaction process is not quickly removed, the reaction heat may accumulate inside the catalyst particles, causing a sintering phenomenon in which the catalyst particles agglomerate, and a deterioration problem in which the activity of the catalyst itself decreases may occur, thereby lowering the selectivity for the target hydrocarbon.

As a method for solving these problems, it is known to control the reactivity of the catalyst layer by using an inert support or inert particles together with the catalyst particles when converting carbon dioxide using a fixed bed reactor, or to use a catalyst with a new structure and characteristics. However, the former has a problem of causing side effects due to the inert support or particles used, and the newly developed catalysts do not show satisfactory results in both the conversion rate of carbon dioxide and the selectivity for high-carbon hydrocarbons at the same time.

Therefore, it is necessary to develop a novel catalyst that can produce hydrocarbons from carbon dioxide without a separate two-stage reaction process while exhibiting satisfactory results in both the conversion rate of carbon dioxide and the selectivity for high-carbon hydrocarbons.

[Prior Art Literature]

(Patent Document 1) KR 10-2023-0040742 A

[Disclosure]

**[Technical Problem]**

The present invention is intended to solve the above-mentioned problem, and aims to provide a novel carbon dioxide conversion catalyst capable of producing high-carbon hydrocarbons, particularly hydrocarbons having 5 or more carbon atoms, with high selectivity while minimizing a sintering phenomenon at high temperatures by introducing a thin and uniform protective layer on the surface of a metal catalyst particle using an atomic layer deposition method and optimizing the ratio of the catalyst particle surface covered by the protective layer.

**[Technical Solution]**

In order to solve the above-mentioned problem, the present invention provides a novel carbon dioxide conversion catalyst, a method for producing the same, and a carbon dioxide conversion method using the catalyst.

Specifically, (1) the present invention provides a catalyst for carbon dioxide conversion, including Fe-based catalyst particles and a protective layer formed on the surface of the catalyst particles, wherein the ratio of the catalyst particle surface covered by the protective layer is 10 to 90%, and the protective layer comprises Al, Ce, Cu, Co, Mo, or an oxide or nitride thereof.

(2) The present invention provides the catalyst for carbon dioxide conversion according to (1) above, wherein the thickness of the protective layer is 0.5 to 10 nm.

(3) The present invention provides the catalyst for carbon dioxide conversion according to (1) or (2) above, wherein the surface area of the catalyst particle surface not covered by the protective layer is 0.05 to 2 $m^2$/g.

(4) The present invention provides the catalyst for carbon dioxide conversion according to any one of (1) to (3) above, wherein the specific surface area of the catalyst is 1 to 250 $m^2$/g.

(5) The present invention provides the catalyst for carbon dioxide conversion according to any one of (1) to (4) above, wherein the content of the protective layer is 1 to 50 wt% based on the weight of the entire catalyst.

(6) The present invention provides the catalyst for carbon dioxide conversion according to any one of (1) to (5) above, wherein the Fe-based catalyst particle is a Fe bulk catalyst, or a catalyst particle in which Fe is supported as an active component on a support.

(7) The present invention provides the catalyst for carbon dioxide conversion according to any one of (1) to (6) above, wherein the Fe-based catalyst particle is a Fe bulk catalyst, and the ratio of the catalyst particle surface covered by the protective layer is 30% or more and 90% or less.

(8) The present invention provides the catalyst for carbon dioxide conversion according to any one of (1) to (7) above, wherein the support is alumina or silica.

(9) The present invention provides the catalyst for carbon dioxide conversion according to any one of (1) to (8) above, wherein the Fe-based catalyst particle is Fe/Si, and the ratio of the catalyst particle surface covered by the protective layer is 10% or more and 20% or less.

(10) The present invention provides the catalyst for carbon dioxide conversion according to any one of (1) to (9) above, wherein the Fe-based catalyst particle is Fe/γ-Al, and the ratio of the catalyst particle surface covered by the protective layer is 5% or more and 15% or less.

(11) The present invention provides the catalyst for carbon dioxide conversion according to any one of (1) to (10) above, wherein the Fe-based catalyst particle is Fe/δ-Al, and the ratio of the catalyst particle surface covered by the protective layer is 20% or more and 30% or less.

(12) The present invention provides the catalyst for carbon dioxide conversion according to any one of (1) to (11) above, wherein the Fe-based catalyst particle is a catalyst particle in which Fe is supported as an active component on a support, and at least one selected from Na, K, and Cu is further supported as a cocatalyst component.

(13) The present invention provides the catalyst for carbon dioxide conversion according to any one of (1) to (12) above, wherein the Fe-based catalyst particle is Fe/K/Cu/Si, and the ratio of the catalyst particle surface covered by the protective layer is 10% or more and 50% or less.

(14) The present invention provides a method for producing the catalyst according to any one of (1) to (13) above, including a step of forming a protective layer on the surface of Fe-based catalyst particles using an atomic layer deposition method.

(15) The present invention provides a method for converting carbon dioxide, the method including a step of synthesizing a mixed hydrocarbon gas by heating a reaction gas containing carbon dioxide in the presence of the catalyst according to any one of (1) to (13) above.

**[Advantageous Effects]**

The carbon dioxide conversion catalyst of the present invention has a structure in which a portion of the surface of a catalyst particle exhibiting catalytic activity is covered by a thin and uniform protective layer, and thus, can exhibit excellent catalytic activity while suppressing a sintering phenomenon at high temperatures, thereby stably and efficiently converting carbon dioxide into hydrocarbons, and in particular, producing a high value-added hydrocarbon having 5 or more carbon atoms among hydrocarbons with high selectivity.

**[Description of Drawing]**

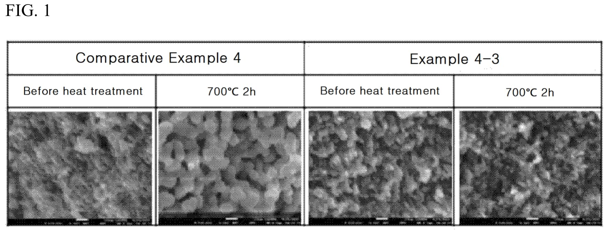

FIG. 1 is a view illustrating changes in the shape of the catalyst of Example 4-3 of the present invention and the catalyst of Comparative Example 4 before and after heat treatment.

**[Best Modes of the Invention]**

Hereinafter, the present invention will be described in more detail.

The terms or words used in the specification and claims of the present application should not be construed as being limited to their ordinary or dictionary meanings, but should be interpreted as meanings and concepts consistent with the technical spirit of the present invention, based on the principle that the inventor may adequately define the concepts of terms to best describe his or her invention.

Catalyst for carbon dioxide conversion

The present invention provides a catalyst for carbon dioxide conversion including Fe-based catalyst particles and a protective layer formed on the surface of the catalyst particles, wherein the ratio of the catalyst particle surface covered by the protective layer is 10 to 90%, and the protective layer comprises Al, Ce, Cu, Co, Mo, or an oxide or nitride thereof.

As described above, the reaction of converting carbon dioxide into hydrocarbon is performed in a two-step reaction, and Fe-based catalysts are known to exhibit relatively excellent activity in both of these reaction steps. However, when the Fe-based catalyst particles are used as they are, there is a disadvantage that the activity decreases due to sintering of the catalyst during the reaction process, and thus, is not high enough to satisfy any one or more of the selectivity and conversion rates mentioned above. In order to overcome these shortcomings, the present invention introduces a thin and uniform protective layer covering a portion of the surface of Fe-based catalyst particles using atomic layer deposition (ALD), and provides a novel catalyst for carbon dioxide conversion that, through the action of such a protective layer, can increase the high-temperature stability of the catalyst itself while also obtaining high selectivity for high-carbon hydrocarbons having 5 or more carbon atoms, which are relatively high in added value among the finally produced hydrocarbon mixtures.

Hereinafter, the catalyst of the present invention will be described in more detail.

**Fe-based catalyst particles**

Fe-based catalyst particles refer to catalyst particles containing Fe, which have been used in the conventional carbon dioxide conversion reaction. A reaction of converting carbon dioxide into hydrocarbons can be performed by the activity of the Fe-based catalyst particles.

More specifically, the Fe-based catalyst particle may be a Fe bulk catalyst, or a catalyst particle in which Fe is supported as an active component on a support. The Fe bulk catalyst refers to a catalyst composed only of Fe-based components as an active component without a separate support. More specifically, Fe metal or a compound containing Fe may be used as the bulk catalyst.

Meanwhile, when the Fe-based catalyst particle is a catalyst particle in which Fe is supported as an active component on a support, the support may be alumina or silica. The above types of supports have a large specific surface area and thus are advantageous for supporting active components, and can be particularly excellent in terms of catalytic activity and durability when Fe is supported.

Furthermore, when the Fe-based catalyst particle is a catalyst particle in which Fe is supported as an active component on a support, a cocatalyst component may be supported together with the active component Fe for the purpose of further increasing the catalytic activity of the active component, and at least one selected from Na, K, and Cu may be used as the cocatalyst component.

The Fe-based catalyst particles may be in the form of secondary particles in which a plurality of primary particles are agglomerated. The primary particles may be nanoparticles having a diameter in the nm unit, and more specifically, the diameter of the primary particles may be 100 nm or less, and particularly preferably 80 nm or less, 60 nm or less, 50 nm or less, 40 nm or less, or 30 nm or less, and also 1 nm or more, 3 nm or more, 5 nm or more, or 10 nm or more. In addition, the diameter of the secondary particles formed by agglomeration of a plurality of the above primary particles may be 10 µm or more, and more preferably 30 µm or more, 50 µm or more, 70 µm or more, or 100 µm or more, and also 1,000 µm or less, 800 µm or less, 600 µm or less, or 500 µm or less. When the diameters of the primary particles and the secondary particles of the Fe-based catalyst particles are within the above-described range, the balance in terms of mechanical strength and catalytic activity may be appropriate.

**Protective layer**

The protective layer serves to suppress catalyst sintering during the carbon dioxide conversion reaction process by covering a portion of the surface of the catalyst particles described above, thereby improving the conversion rate of carbon dioxide and the selectivity for high-carbon hydrocarbons.

More specifically, when the protective layer is formed on the surface of the catalyst particles described above using the atomic layer deposition method, the protective layer is formed in a shape that covers only a portion of the surface of the catalyst particles while having a thin and uniform thickness. When the ratio of the surface of the catalyst particles covered by the protective layer formed in this way, i.e., the coverage rate, is adjusted to a range of 10 to 90%, the conversion rate of carbon dioxide by the catalyst is improved, while the selectivity for high-carbon hydrocarbons also increases. Although the specific mechanism of this action cannot be clearly explained, it was expected that the selectivity would decrease because the surface area exhibiting activity is narrowed when the catalyst surface is covered by the protective layer. However, when the coverage rate is within a specific range as in the present invention, it was confirmed that the selectivity is also improved along with the conversion rate that is expected to be improved by the protective layer.

In addition, the coverage rate may vary depending on the type or component of the Fe-based catalyst particles described above. More specifically, when the internal pore size of the Fe-based catalyst particles is small, the coverage rate may decrease as some of the pores are blocked during the protective layer formation process, and conversely, when the internal pore size of the Fe-based catalyst particles is large, the coverage rate may be high after the protective layer is formed. Furthermore, the coverage rate may be adjusted by changing the process conditions during the protective layer formation process. More specifically, when the protective layer is formed thick, the coverage rate may decrease as the thickened protective layer blocks some of the pores, and when the protective layer is formed thin, the coverage rate may be adjusted within a high range.

The coverage rate may vary depending on the type and component of the Fe-based catalyst particles as described above, and may be 10 to 90%, and when the Fe-based catalyst particles include a support, may be 10% or more and 90% or less, 80% or less, 70% or less, 60% or less, 50% or less, 40% or less, or 30%. Meanwhile, when the Fe-based catalyst particle is a bulk catalyst that does not include a support, it may be 10% or more, 20% or more, 30% or more, or 35% or more and 90% or less. More specifically, when the Fe-based catalyst particle is Fe/Si, the coverage rate may be in the range of 10% or more and 20% or less. When the Fe-based catalyst particle is Fe/$\gamma$-Al, the coverage rate may be in the range of 5% or more and 15% or less. When the Fe-based catalyst particle is Fe/$\delta$-Al, the coverage rate may be in the range of 20% or more and 30% or less. When the Fe-based catalyst particle is Fe/K/Cu/Si, the coverage rate may be in the range of 10% or more and 50% or less, and preferably 10% or more and 50% or less, 45% or less, 40% or less, 35% or less, or 30% or less. When the coverage rate is within the above-described range, the selectivity for high-carbon hydrocarbons and the conversion rate of carbon dioxide can be excellent at the same time.

Meanwhile, the "coverage rate" can be calculated by dividing the content of the protective layer by (protective layer (covering layer) thickness * catalyst specific surface area * theoretical true density of the protective layer). In the above formula, the theoretical true density of the protective layer means a theoretical value known as the density of the component formed as the protective layer.

In addition, the protective layer may include Al, Ce, Cu, Co, Mo, or oxides or nitrides thereof. The above components do not lower the catalytic activity of the Fe-based catalyst particles, and have excellent high-temperature and mechanical stability, so that the performance can be sufficiently maintained even when the catalyst is used under high-temperature conditions. More preferably, the protective layer may include Al. When the protective layer includes Al, or an oxide or nitride thereof, the performance of the above-described protective layer can be maximized, and in particular, the catalytic activity can be enhanced due to the surface interaction between the Fe-based catalyst particles and Al of the protective layer.

Furthermore, the thickness of the protective layer may be 0.5 to 10 nm, and preferably 0.5 nm or more, 1 nm or more, 1.5 nm or more, or 2 nm or more, and 10 nm or less, 9 nm or less, 8 nm or less, 7 nm or less, 6 nm or less, or 5 nm or less. If the thickness of the protective layer is too thin, the effect of the protective layer, specifically, the effect of suppressing the sintering of the catalyst at high temperatures, may not be significantly exerted, and if the thickness of the protective layer is too thick, the catalytic activity may rather decrease. Meanwhile, in the present invention, the thickness of the protective layer can be controlled within the above-mentioned thin range while being uniform because the protective layer is formed using an atomic layer deposition method. The thickness of the protective layer can be measured from a cross-sectional TEM of the manufactured catalyst.

In the catalyst of the present invention, the surface area of the catalyst particle surface not covered by the protective layer may be 0.05 to 2 $m^2/g$, preferably 0.05 $m^2/g$ or more, 0.07 $m^2/g$ or more, or 0.1 $m^2/g$ or more, and also 2 $m^2/g$ or less, 1.8 $m^2/g$ or less, or 1.5 $m^2/g$ or less. A sufficient degree of catalytic activity can be provided only when the surface area of the catalyst particle not covered by the protective layer is within the above-mentioned range.

In the catalyst of the present invention, the specific surface area of the catalyst after the protective layer is formed may vary depending on the type of Fe-based catalyst particles, but may be 1 to 250 $m^2$/g, and preferably 1 to 100 $m^2$/g. The catalyst of the present invention has a sufficiently wide specific surface area as described above, so that the contact area between the catalyst and carbon dioxide can be sufficiently wide, and thus the carbon dioxide conversion rate can be high.

In the catalyst of the present invention, the content of the protective layer based on the weight of the entire catalyst may be 1 to 50 wt%, and preferably 2 wt% or more, 3 wt% or more, 5 wt% or more, or 10 wt% or more, and 30 wt% or less, 25 wt% or less, or 23 wt% or less. When the content of the protective layer is within the above-described range, the improvement effect by the protective layer can be maximized.

### Method of manufacturing catalysts

The present invention provides a method for manufacturing the catalyst for carbon dioxide conversion as described above. Specifically, the present invention provides a method for manufacturing a catalyst, including a step of forming a protective layer on the surface of Fe-based catalyst particles using an atomic layer deposition method.

The protective layer, which is an important technical feature of the catalyst of the present invention, is characterized by being formed using an atomic layer deposition method. When using the atomic layer deposition method, a protective layer of uniform thickness can be formed even for complex 3D Fe-based catalyst particles, and the thickness of the protective layer can also be adjusted to be thin.

The process of forming the protective layer using the atomic layer deposition method may be performed by a conventional method, and may be performed by repeating multiple cycles, wherein each of the multiple cycles includes a process of loading Fe-based catalyst particles into an atomic layer deposition facility, injecting and depositing a protective layer raw material, an oxidation or reduction process, and a purging process. In the above process, water ($H_2O$), ozone ($O_3$), or oxygen ($O_2$) plasma may be injected to form a metal oxide, and hydrogen gas may be injected to form a metal layer.

### Method for converting carbon dioxide

The present invention provides a method for converting carbon dioxide using the catalyst described above. More specifically, the present invention provides a method for converting carbon dioxide, the method including a step of synthesizing a mixed hydrocarbon gas by heating a reaction gas containing carbon dioxide in the presence of the above catalyst.

The selectivity for high-carbon hydrocarbons having 5 or more carbon atoms in the mixed hydrocarbon gas produced using the catalyst of the present invention may be 40% or more, preferably 45% or more. The catalyst of the present invention is characterized in that, by introducing the protective layer described above, the conversion rate of carbon dioxide and the selectivity for high-carbon hydrocarbons are simultaneously high when performing a carbon dioxide conversion reaction using the catalyst.

The conversion reaction may be performed without any particular limitation under conditions applicable to a thermochemical conversion reaction of carbon dioxide, and the reactor used in the present invention is not particularly limited. Furthermore, the temperature at which the reaction is performed is also not particularly limited as long as it is a temperature sufficient for carbon dioxide to be converted. The catalyst of the present invention can minimize a sintering phenomenon even under high-temperature conditions by having a protective layer, so that the carbon dioxide conversion reaction can proceed stably even at relatively high temperatures.

Hereinafter, the present invention will be described in more detail by way of examples and experimental examples to specifically illustrate the present invention, but the present invention is not limited to these examples and experimental examples. However, the examples according to the present invention may be modified in various different forms, and the scope of the present invention should not be construed as being limited to the examples described in detail below. The examples of the present invention are provided to explain the present invention more completely to those skilled in the art.

### Materials

Fe/Si, Fe/$\gamma$-Al, Fe/$\delta$-Al, Fe bulk catalyst, Fe/Na, and Fe/K/Cu/Si catalysts were used as Fe-based catalyst particles. The Fe/Na catalyst refers to a catalyst in which Na, a cocatalyst component, is added to the Fe bulk catalyst, and the Fe/K/Cu/Si catalyst refers to a catalyst in which Fe is supported as a main catalyst component on a Si support, and Cu and K components are supported as cocatalyst components.

**Examples and Comparative Examples**

0.5 g of the prepared Fe-based catalyst particles were applied and loaded onto a square tray. Then, the tray was placed in a chamber in an ALD device, and the chamber was set to have a temperature of 150°C and a process pressure of 1 Torr. Thereafter, a process was repeated 3 times in which trimethylaluminum (TMA) at 20°C was pulse-injected into the chamber together with 100 sccm of nitrogen gas as a carrier gas for 3 seconds, maintained for 100 seconds, and then purged for 20 seconds. Thereafter, a process was repeated 3 times again in which purging was performed again for 600 seconds, and instead of trimethylaluminum in the previous process, water was pulse-injected for 3 seconds in the same manner, maintained for 100 seconds, and then purged for 20 seconds. Taking these processes as one cycle, the cycle was repeated 8 to 40 times to form a protective layer including aluminum oxide on the surface of the Fe-based catalyst particles.

The details of each catalyst manufactured in the examples and comparative examples are summarized in Tables 1 and 2 below. Meanwhile, in the case of Example 5-2, the amount of TMA and water added was doubled, but other conditions were the same as in Example 5-1, so that a catalyst having the same thickness of the protective layer but a higher coverage rate was manufactured. In Example 5-3, the catalyst particles were pulverized and classified to reduce the particle size from the existing 300 to 500 μm to 100 to 300 μm, and then a protective layer was formed. In the case of Example 5-3, as the size of the catalyst particles decreased, the exposed surface area increased, and thus the coverage rate of the protective layer increased.

[Table 1]

|  | Comparative Example 1 | Example 1 | Comparative Example 2 | Example 2 | Comparative Example 3 | Example 3 |
|---|---|---|---|---|---|---|
| Type of Fe-based catalyst particles | Fe/Si | | Fe/γ-Al | | Fe/δ-Al | |
| Coverage rate (%) | - | 14 | - | 11 | - | 24 |
| Protective layer thickness (nm) | - | 5 | - | 5 | - | 5 |
| Protective layer (Al) content (wt%) | - | 23 | - | 13 | - | 18.9 |
| Catalyst specific surface area (m$^2$/g) | 208 | 29 | 144 | 2.26 | 97.3 | 13.7 |
| Fe surface area (m$^2$/g) | 0.0211 | 0.104 | 0.4178 | 0.1528 | Not measured | Not measured |

[Table 2]

|  | Comparative Example 4 | Example 4-1 | Example 4-2 | Example 4-3 | Comparative Example 5 | Example 5-1 | Example 5-2 | Example 5-3 |
|---|---|---|---|---|---|---|---|---|
| Type of Fe-based catalyst particles | Fe bulk catalyst | | | | Fe/K/Cu/Si | | | |
| Coverage rate (%) | - | 87 | 78 | 39 | - | 11.7 | 16.7 | 29.8 |
| Protective layer thickness (nm) | - | 1 | 2 | 5 | - | 2 | 2 | 2 |
| Protective layer (Al) content (wt%) | - | 5.4 | 9.7 | 12 | - | 2.1 | 2.9 | 5.4 |
| Catalyst specific surface area (m$^2$/g) | 37.8 | 32.3 | 28.6 | 24.0 | 71.4 | 69.8 | 66.8 | 70.4 |

(continued)

|  | Comparative Example 4 | Example 4-1 | Example 4-2 | Example 4-3 | Comparative Example 5 | Example 5-1 | Example 5-2 | Example 5-3 |
|---|---|---|---|---|---|---|---|---|
| Fe | 0.0331 | 0.1357 | 0.1401 | 0.0786 | Not | Not | Not | Not |
| surface area ($m^2$/g) |  |  |  |  | measured | measured | measured | measured |

Meanwhile, the characteristics measured in Tables 1 and 2 above were measured by the following methods.

1) Protective layer thickness: Platinum (Pt) or carbon was deposited (sputtered) with a thickness of 100 nm on the protective layer of the catalyst particles, and then a cross-sectional specimen was prepared using focused ion beam (FIB) or $Ar^+$ ion milling. The thickness of the catalyst layer was measured by TEM analysis of the prepared specimen.

2) Protective layer content: The catalyst content was confirmed through ICP component analysis, and 0.1 g of the catalyst particles were dissolved in 1 ml of hydrochloric acid, diluted 10 times, and then ICP-OES analysis was performed. Meanwhile, the protective layer content refers to the Al content included only in the protective layer, excluding the Al content derived from the support.

Measurement equipment: ICP-OES Agilent 5110
Measurement conditions: RF Power 1300 W, Torch Height 15 mm, Plasma gas flow 15 L/min, Sample Gas flow 0.8 L/min, Aux. Gas flow 0.2 L/min, Pump Speed 1.5 ml/min

3) Coverage rate: It was calculated using the protective layer content, protective layer thickness, catalyst specific surface area, and catalyst density as measured previously, and was calculated using the following equation:

Coverage rate = Catalyst content/(Protective layer thickness * Catalyst specific surface area * Theoretical true density of catalyst)

4) Fe surface area: The catalyst was reduced by heat treatment at 400°C for 3 hours, and then CO gas was injected into the reactor to measure the surface area adsorbed. On the other hand, if the Fe surface area decreases even though the protective layer increases, it is confirmed that the Fe surface area decreases as the catalyst particles agglomerate during the reduction process.

Referring to the results in Tables 1 and 2 above, it was confirmed that the Fe bulk catalyst with a relatively large internal pore size showed a higher coverage rate than other catalysts, and some pores were blocked as the thickness of the protective layer increased, thereby reducing the coverage rate. On the other hand, in the case of the remaining catalysts using the support, it was confirmed that as the internal pore size became relatively small, the coverage rate was lower than in the case of using the bulk catalyst. Also in all types of catalysts, it was confirmed that as the formed protective layer blocked some pores, the specific surface area of the catalyst decreased compared to the catalyst without the protective layer formed.

**Experimental Example 1. Measurement of conversion rate and selectivity in carbon dioxide conversion reaction using catalyst**

Mixed hydrocarbons were prepared from carbon dioxide using the catalysts prepared in Examples 5-1 to 5-3 and Comparative Example 5. Specifically, the prepared catalysts were placed in a reactor, and then were pretreated by flowing 5% $CO/N_2$ (250 sccm) for 3 hours while heating to 500°C. Thereafter, the temperature in the reactor was lowered to 350°C, and 25% $CO_2/H_2$ (75 sccm) was flowed and reacted for 6 hours under a pressure condition of 20 bar.

The reaction product formed as a result of the reaction was passed through a constant temperature water bath trap set to 0°C to collect liquid hydrocarbons having 5 or more carbon atoms, and the gaseous product was analyzed in real time using gas chromatography. The reaction was performed continuously for 48 hours or more.

The carbon dioxide conversion rate and the selectivity for hydrocarbons having 5 or more carbon atoms were measured/calculated using the following methods.

1) Conversion rate: calculated using the following equation:

Carbon dioxide conversion rate = (input $CO_2$ flow rate - discharge $CO_2$ flow rate) /(input $CO_2$ flow rate) * 100%

2) Selectivity: calculated using the following equation:

Selectivity for gaseous products = ($C_AH_B$ flow rate * A)/(input $CO_2$ flow rate - discharge $CO_2$ flow rate) * 100%

Selectivity for liquid hydrocarbons with 5 or more carbon atoms = 100% - (total selectivity for gaseous products)

The measured conversion rate and selectivity values are summarized in Table 3 below.

[Table 3]

|  | $CO_2$ Conversion rate (%) | C5+ selectivity (%) |
| --- | --- | --- |
| Example 5-1 | 44.9 | 56.8 |
| Example 5-2 | 46.1 | 57.6 |
| Example 5-3 | 47.8 | 61.8 |
| Comparative Example 5 | 40.0 | 50.0 |

As can be seen from Table 3 above, it was confirmed that when the catalysts of the examples of the present invention were used, the carbon dioxide conversion rate and the selectivity for liquid hydrocarbons with 5 or more carbon atoms having high added value were higher than when the catalysts of the comparative examples were used. Furthermore, in the case of Examples 5-2 and 5-3, in which the thickness of the protective layer was the same but the aluminum content in the protective layer was higher and the coverage rate was higher accordingly, the conversion rate and selectivity values were confirmed to be higher than in Example 5-1, and through this, it was confirmed that the conversion rate and selectivity could be improved by increasing the coverage rate under the same conditions.

**Experimental Example 2. Confirmation of change in characteristics after heat treatment of catalyst**

It was confirmed how the specific surface area of the catalyst was changed and how particle agglomeration occurred after heat treatment of the catalysts of Comparative Example 4 and Example 4-3.
Specifically, each catalyst was subjected to heat treatment for 2 hours at 700°C, and the results are summarized and shown in FIG. 1.
As can be seen from FIG. 1, the catalyst of Example 4-3 of the present invention showed a small change in specific surface area and a little particle agglomeration although heated under high-temperature conditions for a long time, whereas the catalyst of Comparative Example 4 showed a sintering phenomenon in which particles were agglomerated, and thus the specific surface area was greatly reduced.
From this, it was confirmed that the catalyst of the present invention could stably maintain performance even under high-temperature conditions by having a protective layer covering a portion of the surface.

**Claims**

1. A catalyst for carbon dioxide conversion, including:

   Fe-based catalyst particles; and
   a protective layer formed on the surface of the catalyst particles,
   wherein the ratio of the catalyst particle surface covered by the protective layer is 10 to 90%, and
   the protective layer comprises Al, Ce, Cu, Co, Mo, or an oxide or nitride thereof.

2. The catalyst for carbon dioxide conversion according to claim 1, wherein the thickness of the protective layer is 0.5 to 10 nm.

3. The catalyst for carbon dioxide conversion according to claim 1, wherein the surface area of the catalyst particle surface not covered by the protective layer is 0.05 to 2 $m^2/g$.

4. The catalyst for carbon dioxide conversion according to claim 1, wherein the specific surface area of the catalyst is 1 to 250 $m^2/g$.

5. The catalyst for carbon dioxide conversion according to claim 1, wherein the content of the protective layer is 1 to 50 wt% based on the weight of the entire catalyst.

6. The catalyst for carbon dioxide conversion according to claim 1, wherein the Fe-based catalyst particle is a Fe bulk catalyst, or a catalyst particle in which Fe is supported as an active component on a support.

7. The catalyst for carbon dioxide conversion according to claim 6, wherein the Fe-based catalyst particle is a Fe bulk catalyst, and
the ratio of the catalyst particle surface covered by the protective layer is 30% or more and 90% or less.

8. The catalyst for carbon dioxide conversion according to claim 6, wherein the support is alumina or silica.

9. The catalyst for carbon dioxide conversion according to claim 6, wherein the Fe-based catalyst particle is Fe/Si, and
the ratio of the catalyst particle surface covered by the protective layer is 10% or more and 20% or less.

10. The catalyst for carbon dioxide conversion according to claim 6, wherein the Fe-based catalyst particle is Fe/$\gamma$-Al, and
the ratio of the catalyst particle surface covered by the protective layer is 5% or more and 15% or less.

11. The catalyst for carbon dioxide conversion according to claim 6, wherein the Fe-based catalyst particle is Fe/$\delta$-Al, and
the ratio of the catalyst particle surface covered by the protective layer is 20% or more and 30% or less.

12. The catalyst for carbon dioxide conversion according to claim 6, wherein the Fe-based catalyst particle is a catalyst particle in which Fe is supported as an active component on a support, and
at least one selected from Na, K, and Cu is further supported as a cocatalyst component.

13. The catalyst for carbon dioxide conversion according to claim 6, wherein the Fe-based catalyst particle is Fe/K/Cu/Si, and
the ratio of the catalyst particle surface covered by the protective layer is 10% or more and 50% or less.

14. A method for producing the catalyst of claim 1, the method including a step of forming a protective layer on the surface of Fe-based catalyst particles using an atomic layer deposition method.

15. A method for converting carbon dioxide, the method including a step of synthesizing a mixed hydrocarbon gas by heating a reaction gas containing carbon dioxide in the presence of the catalyst of claim 1.

FIG. 1

| Comparative Example 4 | | Example 4-3 | |
|---|---|---|---|
| Before heat treatment | 700°C 2h | Before heat treatment | 700°C 2h |
| | | | |

## INTERNATIONAL SEARCH REPORT

| International application No. |
|---|
| **PCT/KR2024/013722** |

### A.    CLASSIFICATION OF SUBJECT MATTER

**B01J 35/30**(2024.01)i; **B01J 35/61**(2024.01)i; **B01J 23/78**(2006.01)i; **B01J 37/02**(2006.01)i; **C07C 1/12**(2006.01)i; **C10G 2/00**(2006.01)i

According to International Patent Classification (IPC) or to both national classification and IPC

### B.    FIELDS SEARCHED

Minimum documentation searched (classification system followed by classification symbols)

B01J 35/30(2024.01); B01J 19/00(2006.01); B01J 21/04(2006.01); B01J 23/745(2006.01); B01J 23/755(2006.01); B01J 23/89(2006.01); B01J 35/00(2006.01); C01B 32/162(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 촉매(catalyst), 이산화탄소(carbon dioxide), 보호층(protective layer), 원자막 증착법(atomic layer deposition)

### C.    DOCUMENTS CONSIDERED TO BE RELEVANT

| Category* | Citation of document, with indication, where appropriate, of the relevant passages | Relevant to claim No. |
|---|---|---|
| DY | KR 10-2023-0040742 A (KOREA RESEARCH INSTITUTE OF CHEMICAL TECHNOLOGY) 23 March 2023 (2023-03-23)<br>See claim 1; and paragraphs [0057]-[0065] and [0108]-[0113]. | 1-15 |
| Y | CN 107754814 B (JIANGNAN UNIVERSITY) 04 September 2020 (2020-09-04)<br>See abstract; and claim 1. | 1-15 |
| Y | KR 10-2016-0092547 A (KOREA UNIVERSITY OF TECHNOLOGY AND EDUCATION INDUSTRY-UNIVERSITY COOPERATION FOUNDATION) 05 August 2016 (2016-08-05)<br>See claim 1. | 9-13 |
| A | US 2012-0329889 A1 (YANG, J. et al.) 27 December 2012 (2012-12-27)<br>See claims 1-17. | 1-15 |
| A | KR 10-2023-0109576 A (LG CHEM, LTD.) 20 July 2023 (2023-07-20)<br>See claims 1-10. | 1-15 |

☐ Further documents are listed in the continuation of Box C.      ☑ See patent family annex.

| | |
|---|---|
| *    Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **19 December 2024** | **19 December 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.

**PCT/KR2024/013722**

| Patent document cited in search report | | | Publication date (day/month/year) | Patent family member(s) | | | Publication date (day/month/year) |
|---|---|---|---|---|---|---|---|
| KR | 10-2023-0040742 | A | 23 March 2023 | KR | 10-2572062 | B1 | 29 August 2023 |
| CN | 107754814 | B | 04 September 2020 | CN | 107754814 | A | 06 March 2018 |
| KR | 10-2016-0092547 | A | 05 August 2016 | None | | | |
| US | 2012-0329889 | A1 | 27 December 2012 | EP | 2537581 | A1 | 26 December 2012 |
| | | | | EP | 2537581 | A4 | 14 December 2016 |
| | | | | EP | 2537581 | B1 | 30 September 2020 |
| | | | | KR | 10-1094077 | B1 | 15 December 2011 |
| | | | | KR | 10-2011-0094462 | A | 24 August 2011 |
| | | | | US | 10160916 | B2 | 25 December 2018 |
| | | | | US | 10160917 | B2 | 25 December 2018 |
| | | | | US | 2016-0200985 | A1 | 14 July 2016 |
| | | | | US | 2016-0200986 | A1 | 14 July 2016 |
| | | | | WO | 2011-102567 | A1 | 25 August 2011 |
| KR | 10-2023-0109576 | A | 20 July 2023 | CN | 117561119 | A | 13 February 2024 |

Form PCT/ISA/210 (patent family annex) (July 2022)

**REFERENCES CITED IN THE DESCRIPTION**

**Patent documents cited in the description**

- KR 1020230123490
- KR 1020230040742 A